(19) Europäisches Patentamt
European Patent Office
Office européen des brevets

(11) Veröffentlichungsnummer: **0 337 947**
**A1**

# (12) EUROPÄISCHE PATENTANMELDUNG

(21) Anmeldenummer: **89810255.3**

(22) Anmeldetag: **04.04.89**

(51) Int. Cl.[4]: **C 07 D 487/04**
C 07 D 327/04,
C 07 D 327/06, A 01 N 43/90
//(C07D487/04,249:00,239:00),
(C07D487/04,251:00,249:00)

(30) Priorität: **13.04.88 CH 1356/88**

(43) Veröffentlichungstag der Anmeldung:
**18.10.89 Patentblatt 89/42**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Föry, Werner, Dr.**
**Inzlingerstrasse 11**
**CH-4125 Riehen (CH)**

Patentansprüche für folgenden Vertragsstaat: ES.

(54) **Triazolylsulfonamide.**

(57) N-Benzoxathiolanyl- und N-Benzoxathiinyl-triazolopyrimidinyl- und -triazolotriazinylsulfonamide der Formel

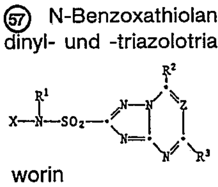

worin
X einen Rest der Formel

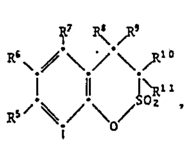,

x1

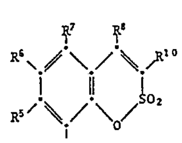

x2

oder

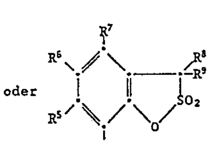

x3

Z Stickstoff oder $CR^4$,
$R^1$ Wasserstoff, $C_1$-$C_6$-Alkyl, durch Halogen, $C_1$-$C_4$-Alkoxycarbonyl, $C_2$-$C_6$-Alkenyl oder $C_2$-$C_6$-Alkinyl substituiertes $C_1$-$C_6$-Alkyl, Benzyl oder durch Halogen, Methyl, Methoxy, -CO-$R^{12}$, -CO-O$R^{12}$, -$SO_2$-$R^{12}$ oder -CO-N$R^{13}R^{14}$ substituiertes Benzyl,
$R^2$, $R^3$ und $R^4$ unabhänig voneinander Wasserstoff, $C_1$-$C_6$-Alkyl, $C_3$-$C_6$-Cycloalkyl, $C_1$-$C_6$-Halogenalkyl, $C_1$-$C_6$-Alkoxy, oder $R^2$ und $R^4$ zusammen oder $R^3$ und $R^4$ zusammen eine $C_3$-$C_4$-Alkylenkette oder eine $C_2$-$C_4$-Alkylenkette, worin ein Kettenglied durch Sauerstoff, Schwefel, -SO-, -$SO_2$-, -NH- oder -N($C_1$-$C_4$-Alkyl)- ersetzt sein kann,
$R^5$, $R^6$ und $R^7$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkoxycarbonyl, $C_2$-$C_4$-Alkoxyalkoxy oder -CO-N$R^{15}R^{16}$, mit der Massgabe, dass stets einer der Reste $R^5$, $R^6$ und $R^7$ für Wasserstoff steht,
$R^8$, $R^9$, $R^{10}$ und $R^{11}$ unabhängig voneinander Wasserstoff oder $C_1$-$C_4$-Alkyl, oder
$R^8$ und $R^9$ zusammen oder $R^{10}$ und $R^{11}$ zusammen mit dem sie tragenden Kohlenstoffatom einen spirocyclisch verknüpften $C_3$ bis $C_6$-Cycloalkylring bilden,
$R^{12}$ $C_1$-$C_4$-Alkyl, Phenyl, Benzyl, durch Halogen, Methyl oder Trifluoromethyl substituiertes Phenyl oder durch Halogen, Methyl oder Trifluormethyl substituiertes Benzyl, und
$R^{13}$, $R^{14}$, $R^{15}$ und $R^{16}$ unabhängig voneinander Wasserstoff oder $C_1$-$C_4$-Alkyl bedeuten.

EP 0 337 947 A1

## Beschreibung

### Triazolylsulfonamide

Die vorliegende Erfindung betrifft neue, herbizid wirksame N-Benzoxathiolanyl- und N-Benzoxathiinyl-triazolopyrimidinyl- und -triazolotriazinylsulfonamide, Verfahren zu ihrer Herstellung, die sie als Wirkstoffe enthaltende Mittel sowie deren Verwendung zum Bekämpfen von Unkräutern, vor allem selektiv in Nutzpflanzenkulturen.

Die erfindungsgemässen Wirkstoffe entsprechen der Formel I

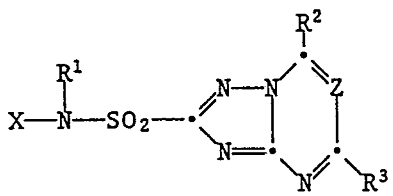

(I)

worin

X einen Rest der Formel

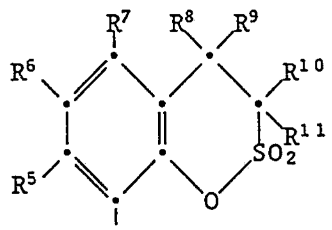

X1

,

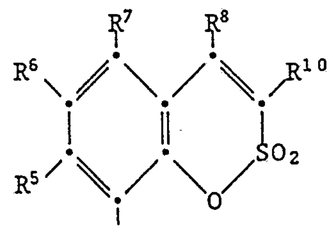

X2

oder

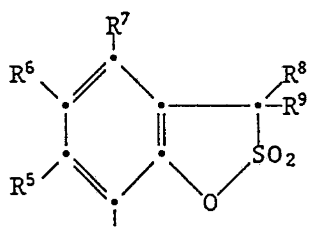

X3

,

Z Stickstoff oder $CR^4$,

$R^1$ Wasserstoff, $C_1$-$C_6$-Alkyl, durch Halogen, $C_1$-$C_4$-Alkoxycarbonyl, $C_2$-$C_6$-Alkenyl oder $C_2$-$C_6$-Alkinyl substituiertes $C_1$-$C_6$-Alkyl, Benzyl oder durch Halogen, Methyl, Methoxy, $-CO-R^{12}$, $-CO-OR^{12}$, $-SO_2-R^{12}$ oder $-CO-NR^{13}R^{14}$ substituiertes Benzyl,

$R^2$, $R^3$ und $R^4$ unabhänig voneinander Wasserstoff, $C_1$-$C_6$-Alkyl, $C_3$-$C_6$-Cycloalkyl, $C_1$-$C_6$-Halogenalkyl, $C_1$-$C_6$-Alkoxy, oder

$R^2$ und $R^4$ zusammen oder $R^3$ und $R^4$ zusammen eine $C_3$-$C_4$-Alkylenkette oder eine $C_2$-$C_4$-Alkylenkette, worin ein Kettenglied durch Sauerstoff, Schwefel, -SO-, $-SO_2-$, -NH- oder $-N(C_1$-$C_4$Alkyl)- ersetzt sein kann,

$R^5$, $R^6$ und $R^7$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkoxycarbonyl, $C_2$-$C_4$-Alkoxyalkoxy oder $-CO-NR^{15}R^{16}$, mit der Massgabe, dass stets einer der Reste $R^5$, $R^6$ und $R^7$ für Wasserstoff steht,

$R^8$, $R^9$, $R^{10}$ und $R^{11}$ unabhängig voneinander Wasserstoff oder $C_1$-$C_4$-Alkyl, oder

$R^8$ und $R^9$ zusammen oder $R^{10}$ und $R^{11}$ zusammen mit dem sie tragenden Kohlenstoffatom einen spirocyclisch verknüpften $C_3$-$C_6$-Cycloalkylring bilden,

$R^{12}$ $C_1$-$C_4$-Alkyl, Phenyl, Benzyl, durch Halogen, Methyl oder Trifluoromethyl substituiertes Phenyl oder durch Halogen, Methyl oder Trifluormethyl substituiertes Benzyl, und

$R^{13}$, $R^{14}$, $R^{15}$ und $R^{16}$ unabhängig voneinander Wasserstoff oder $C_1$-$C_4$-Alkyl bedeuten.

Triazolopyrimidinsulfonamide und Triazolotriazinsulfonamide mit herbizider Wirkung sind aus der Europäischen Patentanmeldung EP-A-142 152 und dem US-Patent 4 605 433 bekannt. Neben einer Herbizidwirkung wird diesen Verbindungen die Fähigkeit zugeschrieben, die Stickstoffwaschung des Ackerbodens zu verzögern.

In den Definitionen ist unter Alkyl geradkettiges oder verzweigtes Alkyl zu verstehen; z.B.: Methyl, Aethyl,

n-Propyl, i-Propyl oder die vier isomeren Butyl, n-Pentyl oder die isomeren Pentyl, Hexyl oder die isomeren Hexyl. Bevorzugt sind Alkylreste mit höchstens 4 Kohlenstoffatomen.

Unter Alkoxy ist zu verstehen: Methoxy, Aethoxy, n-Propyloxy, i-Propyloxy oder die vier isomeren Butyloxy, n-Pentyloxy oder die isomeren Pentyloxy, n-Hexyloxy oder die isomeren Hexyloxy, insbesondere aber Methoxy, Aethoxy oder i-Propyloxy.

Beispiele für Alkylthio sind Methylthio, Aethylthio, n-Propylthio, i-Propylthio oder die vier isomeren Butylthio, insbesondere aber Methylthio und Aethylthio. Die Alkylsulfinyl und Alkylsulfonylreste leiten sich aus den genannten Alkylthiogruppen ab, unterscheiden sich aber in der Oxidationsstufe des Schwefelatoms.

Unter Halogen selbst sowie als Teil eines Substituenten wie in Halogenbenzyl, Halogenphenyl oder Halogenalkyl sind Fluor, Chlor und Brom, vorzugsweise jedoch Fluor und Chlor zu verstehen. Halogenalkyl steht in der Regel für Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Substituierte Phenylgruppen sind beispielsweise: 2-Chlorphenyl, 3-Chlorphenyl, 4-Chlorphenyl, 2,4-Dichlorphenyl, 3,4-Dichlorphenyl, 2,4,6-Trichlorphenyl, 2,5-Dichlorphenyl, 4-Fluorphenyl, 2-Fluorphenyl, 4-Methylphenyl, 2,4-Dimethylphenyl, 4-Trifluormethylphenyl oder 4-Bromphenyl. Substituierte Benzylgruppen besitzen im Rahmen der Erfindung neben den oben aufgezählten Phenylgruppen als aromatische Bestandteile zum Beispiel auch folgende Gruppen: 4-Methoxyphenyl, 2,4-Dimethoxyphenyl, 3-Alkoxycarbonylphenyl, 3-Acetylphenyl, 4-Alkylsulfonylphenyl, 4-(4′-Methylphenylsulfonyl-phenyl oder Dimethylaminocarbonylphenyl.

Beispiele für Alkoxyalkyl sind: Methoxymethyl, Methoxyäthyl, Methoxypropyl, Aethoxyäthyl, Aethoxymethyl oder Propyloxymethyl. Beispiele für Alkoxyalkoxy sind: Methoxymethoxy, Methoxyäthoxy, Methoxypropyloxy, Aethoxymethoxy, Aethoxyäthoxy sowie Propyloxymethoxy.

Alkylenketten sind im Rahmen der Definition unter Formel I Aethylen, Propylen, Butylen, 1-Methyläthylen, 1-Aethyläthylen, 2-Methylbutylen, 1-Methylbutylen oder wenn Kettenglieder durch Sauerstoff, Schwefel, -SO-, $-SO_2-$, -NH- oder $-N(C_1-C_4\text{-Alkyl})$ ersetzt sind, auch $-O-CH_2-CH_2-$, $-CH_2-S-CH_2-CH_2-$, $-O-CH_2-O-$, $-O-CH_2-CH_2-O-$, $-S-CH_2-CH_2-$, $-CH_2-N(CH_3)-CH_2-$, $-S-CH_2-CH_2-CH_2-$, $-CH_2-SO_2-CH_2-CH_2-$, $-CH_2-O-CH_2-$, $-O-CH_2-CH_2-CH_2-$, $-CH_2-O-CH_2-CH_2-$, $-O-CH_2-O-CH_2-$, $-NH-CH_2-CH_2-CH_2-$, $-CH_2-SO-CH_2-$, $-NH-CH_2-CH_2-$ oder $-CH_2-N(C_2H_5)-CH_2-CH_2-$.

Unter Alkenyl ist in den Definitionen geradkettiges oder verzweigtes Alkenyl zu verstehen; wie zum Beispiel: Vinyl, Allyl, 2-Propenyl, Methallyl, 3-Butenyl, 2-Butenyl, 3-Pentenyl, 2-Methyl-4-pentenyl, 3-Hexenyl.

In den Definitionen ist unter Alkinyl geradkettiges oder verzweigtes Alkinyl zu verstehen; z.B.: Propargyl, Aethinyl, 2-Propinyl, 3-Butinyl, 2-Methyl-3-pentinyl, 1,2-Dimethyl-3-butinyl.

Beispiele für Alkoxycarbonyl sind vorzugsweise Methoxycarbonyl, Aethoxycarbonyl oder i-Propyloxycarbonyl.

Cycloalkyl steht für Cyclopropyl, Dimethylcyclopropyl, Cyclobutyl, Cyclopentyl, Methylcyclopentyl oder Cyclohexyl, vorzugsweise aber für Cyclopropyl, Cyclopentyl oder Cyclohexyl.

Die Verbindungen der Formel I, worin $R^1$ für Wasserstoff steht (Formel Ia), zeichnen sich durch einen niedrigen pKa-Wert aus. Sie bilden daher leicht Salze mit Aminen, Alkali- und Erdalkalimetallbasen sowie quaternären Ammoniumbasen.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Aethylamin, Propylamin, i-Propylamin, die vier isomeren Butylamine, Dimethyl amin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin, Diäthanolamin und 1,4-Diazabicyclo[2.2.2}octan.

Beispiele für quaternäre Ammoniumbasen sind im allgemeinen die Kationen von Halogenammoniumsalzen, z.B. das Tetramethylammoniumkation, das Tetraäthylammoniumkation, das Trimethyläthylammoniumkation, aber auch das Ammoniumkation.

Die entsprechenden Salze werden beispielsweise durch Umsetzung der Verbindungen der Formel Ia mit einer äquimolaren Menge Base und Verdampfen des eingesetzten Lösungsmittels hergestellt.

Unter den Verbindungen der Formel I sind diejenigen bevorzugt, in denen entweder

a) der Substituent X für die Gruppe $X^1$ steht, oder

b) der Substituent X für die Gruppe $X^2$ steht, oder

c) $R^1$ Wasserstoff bedeutet, oder

d) das Ringglied Z für $=CR^4-$ steht, wobei $R^4$ Wasserstoff oder Methyl bedeutet, oder

e)$R^2$ und $R^3$ unabhängig voneinander Wasserstoff, Methyl oder Methoxy bedeuten.

Als weitere bevorzugte Untergruppe von Verbindungen der Formel I ist die Gruppe hervorzuheben, worin $R^1$ Wasserstoff, $R^2$ und $R^3$ unabhängig voneinander Wasserstoff, Methyl oder Methoxy und Z das Ringglied $=CH-$oder $=C(CH_3)-$ bedeuten.

Aus den Untergruppen a und b sind jeweils die Verbindungen bevorzugt, worin zwei von $R^5$, $R^6$ und $R^7$ unabhängig voneinander für Wasserstoff, Halogen, $C_1$-$C_4$-Alkyl oder $C_1$-$C_4$-Alkoxy stehen und der dritte Wasserstoff bedeutet. Innerhalb dieser engeren Gruppen zeigt sich eine weitere

Bevorzugung bei solchen Verbindungen, worin zwei von $R^5$, $R^6$ und $R^7$ unabhängig voneinander für Wasserstoff, Methyl, Chlor oder Methoxy stehen und der dritte Wasserstoff bedeutet.

Von den Verbindungen der Untergruppe a sind ganz besonder diejenigen herauszuheben, worin zwei von $R^5$, $R^6$ und $R^7$ unabhängig voneinander für Wasserstoff, Halogen, $C_1$-$C_4$-Alkyl oder $C_1$-$C_4$-Alkoxy stehen, und der dritte Wasserstoff bedeutet, und $R^8$, $R^9$, $R^{10}$ und $R^{11}$ unabhängig voneinander Wasserstoff oder Methyl bedeuten.

Unter den Verbindungen der Untergruppe Q sind diejenigen besonders bevorzugt, worin zwei von $R^5$, $R^6$ und $R^7$ unabhängig voneinander für Wasserstoff, Halogen, $C_1$-$C_4$-Alkyl oder $C_1$-$C_4$-Alkoxy stehen und der dritte Wasserstoff bedeutet, und $R^8$ und $R^{10}$ unabhängig voneinander Wasserstoff oder Methyl bedeuten.

Ganz besondere herausragende Gruppen von Verbindungen der Formel I sind dadurch charakterisiert, dass

aa) X die Gruppe X1, $R^1$ Wasserstoff, $R^2$ und $R^3$ unabhängig voneinander Wasserstoff, Methyl oder Methoxy, zwei von $R^5$, $R^6$ und $R^7$ unabhängig voneinander Wasserstoff, Methyl, Chlor oder Methoxy, der dritte von $R^5$ $R^6$ und $R^7$ Wasserstoff, und $R^8$, $R^9$, $R^{10}$ unabhängig voneinander Wasserstoff oder Methyl bedeuten; oder dass

bb) X die Gruppe X2, $R^1$ Wasserstoff, $R^2$ und $R^3$ unabhängig voneinander Wasserstoff, Methyl oder Methoxy, zwei von $R^5$, $R^6$ und $R^7$ unabhängig voneinander Wasserstoff, Methyl, Chlor oder Methoxy, der dritte von $R^5$, $R^6$ und $R^7$ Wasserstoff, und $R^8$ und $R^{10}$ unabhängig voneinander Wasserstoff oder Methyl bedeuten.

Insbesondere fallen von den Gruppen aa und bb solche Verbindungen durch ihre gute biologische Wirkung ins Auge, worin Z die Gruppe =CH- oder =$C(CH_3)$- bedeutet.

Als bevorzugte Einzelverbindungen aus dem Umfang der Formel I sind zu nennen:

N-(2,2-Dioxo-1,2-benzoxathiin-8-yl)-5,7-dimethyl-1,2,4-triazolo[1,5-a] pyrimidin-2-yl-sulfonamid und N-(3,4-Dihydro-2,2-dioxo-3-methyl-1,2-benzoxathiin-8-yl)-5,7-dimethyl-1,2,4-triazolo[1,5-a] pyrimidin-2-yl-sulfonamid.

Die Verbindungen der Formel I werden hergestellt, indem man ein primäres Amin der Formel II

$X$-$NH_2$ (II)

worin X die unter Formel I gegebene Bedeutung hat, in Gegenwart einer Base mit einem Triazolopyrimidinyl- oder Triazolotriazinylsulfonylchlorid der Formel III

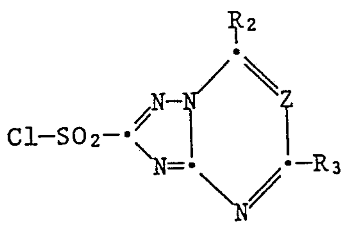

(III)

worin $R^2$, $R^3$ und Z die unter Formel I gegebenen Bedeutungen haben, umsetzt und gewünschtenfalls das erhaltene sekundäre Amin der Unterformel Ia

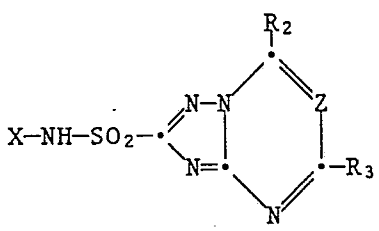

(Ia)

worin $R^2$, $R^3$, X und Z die unter Formel I gegebenen Bedeutungen haben, in Gegenwart einer Base mit einer Verbindung der Formel IV

$R^1$-Y (IV)

worin $R^1$ mit Ausnahme von Wasserstoff die unter Formel I gegebene Bedeutung hat und Y für eine leaving group steht, alkyliert.

Das erfindungsgemässe Verfahren wird mit Vorteil in einem inerten Lösungsmittel bei einer Temperatur zwischen -20°C und der Siedetemperatur des Reaktionsgemisches durchgeführt. Ueblichersweise liegen die Temperaturen zwischen +15°C und +120°C, vorzugsweise zwischen +40°C und +80°C. Geeignete Lösungsmittel sind chlorierte Kohlenwasserstoffe, wie Methylenchlorid, Chloroform, Tetrachlorkohlenstoff, Trichloräthan, Tetrachloräthan, Chlorbenzol oder Dichlorbenzol; aromatische Kohlenwasserstoffe wie Benzol, Toluol oder Xylol; Aether wie Diäthyläther, Diisopropyläther, Tetrahydrofuran, Aethylenglykoldimethyläther oder Dioxan; Nitrile wie Acetonitril, opder Propionitril; Cyclohexan oder Pyridin. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten werden ebenfalls durch Zugabe einiger Tropfen Base als Reaktionskatalysator verkürzt. Als Basen sind insbesondere tertiäre

Amine wie Trimethylamin, Triäthylamin, N-Methylmorpholin, Chinuclidin, Pyridin, 1,4-Diazabicyclo[2.2.2]-octan, 1,5-Diazabicyclo[4.3.0]non-5-en oder 1,8-Diazabicyclo[5.4.0]undec-7-en geeignet. Da Pyridin sowohl als Lösungsmittel, als auch als Katalysator dienen kann, ist es empfehlenswert, die Reaktion in Pyridin auszuführen.

Bei reaktionsträgen Aminen der Formel II empfiehlt es sich, zunächst durch Behandlung mit einer starken Alkalibase das entsprechende Alkaliamid zu erzeugen, und dieses dann mit dem Sulfonylchlorid der Formel III umzusetzen. In diesen Fällen liegt die Reaktionstemperatur deutlich tiefer, vorzugsweise zwischen -80° C und 0° C. Als Lösungsmittel eignen sich hierbei insbesondere Aether wie Diäthyläther oder Tetrahydrofuran. Geeignete Alkalimetallbasen sind: Alkalialkyle wie Butyllithium; und Alkalihydride wie Natrumhydrid, Kaliumhydrid oder Lithiumhydrid. Die in situ erzeugten Alkaliamide der Verbindungen der Formel II können durch Zusatz der Sulfonylchloride der Formel III zu den Endprodukten der Formel Ia umgesetzt werden. Wird eine weitergehende Substitution des an das Stickstoffatom gebundenen Wasserstoffs durch einen von Wasserstoff verschiedenen Rest $R^1$ gewünscht, so kann das Wasserstoffatom mittels eines elektrophilen Reagenzes $R^1$-Y ersetzt werden. Diese Substitution wird ebenfalls durch die katalytische Wirkung einer Base erleichtert. Lösungsmittel, Reaktionstemperaturen und Basen entsprechen denen des obligatorischen Verfahrensschritts (II + III→Ia). Die leaving group Y zeichnet sich durch ihre leichte Substituierbarkeit in einer nucleophilen Substitutionsreaktion aus. Ueblicherweise ist Y ausgewählt aus der Gruppe Halogen, $-O-SO_2-CH_3$, $-O-SO_2-CF_3$, $-O-SO_2-C_6H_5$ oder $-O-SO_2-4-CH_3-C_6H_4$. Für den Fall, dass $R^1$ für eine Acylgruppe $-CO-R^{12}$ steht, kann die leaving group auch für die Gruppe $-O-CO-R^{12}$ stehen.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen, gereinigt werden.

Die Zwischenprodukte der Formel III sind aus EP-A-142 152 und US-PS 4 605 433 bekannt. Die Zwischenprodukte der Formel IV sind bekannt, analog zu bekannten Methoden herstellbar und teilweise im Handel erhältlich.

Die Zwischenprodukte der Formel II sind neu. Sie wurde speziell für die Synthese der neuen Wirkstoffe der Formel I entwickelt. Sie bilden daher einen Bestandteil der vorliegenden Erfindung.

Die primären Amine der Formel II können durch Reduktionsverfahren aus den entsprechenden Nitroverbindungen der Formel V

$X-NO_2$ (V)

worin X die unter Formel I gegebene Bedeutung hat, erhalten werden.

Zur Reduktion der Nitroverbindungen der Formel V zu den Aminoverbindungen der Formel II sind alle gängigen in der Literatur beschriebenen Verfahren einsetzbar. Beispielsweise lässt sich die Reduktion mit Vorteil in wässrigem Medium in Gegenwart von Eisen, Zinn oder Zink und Salzsäure durchführen. Weitere geeignete Methoden sind Reduktionsverfahren unter Verwendung komplexer Hydride wie Lithiumaluminiumhydrid oder die katalytische Reduktion mit Wasserstoff unter Zuhilfenahme von Platin-, Palladium- oder Nickelkatalysatoren.

Die Zwischenprodukte der Formel V sind ebenfalls neu, unter Ausnahme der Verbindung 5-Brom-7-nitro-2,2-dioxo-benzoxathiolan, welche in J.A.C.S. 100 (1978) p. 2525-2534 beschrieben wurde. Diese neuen Verbindungen wurden im Rahmen der vorliegenden Erfindung speziell für die Synthese der neuen Wirkstoffe der Formel I entwickelt und hergestellt. Sie bilden daher einen weiteren Aspekt der vorliegenden Erfindung.

Die Nitroverbindungen der Formel V können durch elektrophile Substitution in für aromatische Verbindungen üblichen Nitrierungsverfahren hergestellt aus den Verbindungen der Formel VI

X-H (VI)

werden. Gewünschtenfalls lassen sich Untergruppen der Formel V, worin X für X1 steht, auch durch selektive Reduktion von Untergruppen der Formel V, worin X für X2 steht, herstellen. Die selektive Reduktion der Doppelbindung im Heterocyclus lässt sich beispielsweise mit Boranen vorteilhaft durchführen. Ferner besteht die Möglichkeit, Aminoverbindungen der Formel II, worin X für X1 steht, aus Nitroverbindungen der Formel V, worin X für X2 steht, durch Reduktion der Aminogruppe bei gleichzeitiger Hydrierung der Doppelbindung im Heterocyclus zu erhalten. Beispielsweise lässt sich diese Methode vorteilhaft mit Hilfe von Platin-oder Palladiumkatalysatoren in Gegenwart von Wasserstoff durchführen. Da als Ausgangsverbindungen für die erfindungsgemässen Wirkstoffe der Formel I nur solche nitrierten Verbindungen in Frage kommen, bei denen die Nitrogruppe die ortho-Position zur Sauerstoff-Bindung im Molekülteil X besetzt, empfiehlt es sich, die entsprechende para-Position vor der Nitrierung zu schützen. Als Schutzgruppe kommt beispielsweise ein Halogenatom in Frage, welches sich gewünschtenfalls mittels katalytischer Reduktion wieder durch ein Wasserstoffatom ersetzen lässt. Als Nitrierungsreagenzien kommen beispielsweise Salpetersäure, Gemische vom Salpetersäure mit Schwefelsäure oder Essigsäure, Stickstoffpentoxid oder Gemische von Salpetersäure mit Stickstoffpentoxid in Frage.

Die Wirkstoffe der Formel I sind stabile Verbindungen. Ihre Handhabung bedarf keiner vorsorglichen Massnahmen.

Die Verbindungen der Formel I besitzen eine ausgeprägte herbizide Wirkung und können daher zur Bekämpfung unerwünschten Pflanzenwuchses verwendet werden. Die Aufwandmengen liegen in der Regel im Bereich von 0,001 bis 1,0 kg AS/ha. Innerhalb des vorgenannten Bereichs wirken die Verbindungen der Formel I bei niedrigeren Aufwandmengen in erster Linie gegen Unkräuter wie beispielsweise Xanthium, Amaranthus,

Chenopodium, Solanum, Sinapis, Stellaria, Chrysanthemum, Galium, Veronica, Sorghum halepense, Avena, Bromus, während Kulturpflanzen wie Gerste, Weizen, Mais, Sorghum, Baumwolle, Soja nicht beeinträchtigt werden. Die Verbindungen der Formel I können daher insbesondere zur selektiven Bekämpfung von Unkräutern in Nutzpflanzenkulturen wie Gerste, Weizen, Mais, Baumwolle, Soja verwendet werden.
Bei Aufwandmengen von 0,015 bis 0,25 kg AS/ha sind die Verbindungen der Formel I beispielsweise zur selektiven Bekämpfung von Unkräutern in Kulturen von Gerste, Weizen, Mais, Baumwolle, Soja geeignet wobei sie ihre Wirkung insbesondere gegen monokotyledone Unkräuter wie Sorghum halepense und dikotyledone Unkräuter wie Xanthium, Amaranthus, Chrysanthemum, Chenopodium, Sinapis, Stellaria, Galium, Veronica entfalten.
Bei höheren Aufwandmengen zeigen die Verbindungen der Formel I totalherbizide Wirkung. Sie können daher zur vollständigen Beseitigung unerwünschten Pflanzenwuchses verwendet werden.

Die Erfindung betrifft auch herbizide Mittel, welche einen neuen Wirkstoff der Formel I enthalten, sowie Verfahren zur pre- und postemergenten Unkrautbekämpfung von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfonate liegen in der Regel als Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen

und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyäthylenglykoläther, Polypropylen-polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 % insbesondere 0,1 bis 25 % eines Tensides.

Insbesondere setzen sich bevorzugte Formulierungen folgendermassen zusammen: (% = Gewichtsprozent).

Emulgierbare Konzentrate:

| | | | |
|---|---|---|---|
| Aktiver Wirkstoff: | 1 bis 20 %, | bevorzugt | 5 bis 10 % |
| oberflächenaktive Mittel: | 5 bis 30 %, | vorzugsweise | 10 bis 20 % |
| flüssiges Trägermittel: | 50 bis 94 %, | vorzugsweise | 70 bis 85 % |

Stäube:

| | | | |
|---|---|---|---|
| Aktiver Wirkstoff: | 0,1 bis 10 %, | vorzugsweise | 0,1 bis 1 % |
| festes Trägermittel: | 99,9 bis 90 %, | vorzugsweise | 99,9 bis 99 % |

Suspensions-Konzentrate:

| | | | |
|---|---|---|---|
| Aktiver Wirkstoff: | 5 bis 75 %, | vorzugsweise | 10 bis 50 % |
| Wasser: | 94 bis 24 %, | vorzugsweise | 88 bis 30 % |
| oberflächenaktives Mittel: | 1 bis 40 %, | vorzugsweise | 2 bis 30 % |

Benetzbares Pulver:

| | | | |
|---|---|---|---|
| Aktiver Wirkstoff: | 0,5 bis 90 %, | vorzugsweise | 1 bis 80 % |
| oberflächenaktives Mittel: | 0,5 bis 20 %, | vorzugsweise | 1 bis 15 % |
| festes Trägermittel: | 5 bis 99 %, | vorzugsweise | 15 bis 90 % |

Granulate:

| | | | |
|---|---|---|---|
| Aktiver Wirkstoff: | 0,5 bis 30 %, | vorzugsweise | 3 bis 15 % |
| festes Trägermittel: | 99,5 bis 70 %, | vorzugsweise | 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

Beispiel H1: N-(2,2-Dioxo-1,2-benzoxathiin-8-yl)-5,7-dimethyl-1,2,4-triazolo[1,5-a] pyrimidin-2-yl-sulfonamid (Verbindung 2.05)

a) 2-Hydroxy-3-nitrobenzaldehyd

Zu einer Lösung von 260 g Aluminiumnitrat $Al(NO_3)_3 \cdot 9H_2O$ in 400 ml Wasser wird eine Lösung von 220 ml 2-Hydroxybenzaldehyd gelöst in 3,3 Liter Eisessig gegeben. Zu dieser Reaktionsmischung lässt man bei 20-25°C innerhalb von 30 Minuten 250 ml konzentrierte Salpetersäure zutropfen. Nach fünfstündigem Rühren bei Raumtemperatur wird die Lösung in 8 Liter Eiswasser eingerührt. Der entstandene Niederschlag wird abfiltriert, mit Wasser gewaschen und bei 40°C getrocknet. Man erhält 277,7 g eines Gemisches aus 2-Hydroxy-3-nitrobenzaldehyd und 2-Hydroxy-5-nitrobenzaldehyd mit einem Schmelzpunkt von 83-84 °C, welches ohne weitere Reinigung weiterverarbeitet wird.

b) 2-Methylsulfonyloxy-3-nitrobenzaldehyd

Zu einer Lösung von 250,5 g eines Gemisches aus 2-Hydroxy-3-nitrobenzaldehyd und 2-Hydroxy-5-nitrobenzaldehyd in 2,5 Liter Aethylacetat und 346,5 ml Triäthylanilin werden bei 0 bis 5° C innerhalb von 25 Minuten 175,5 ml Methylsulfonylchlorid zugetropft. Nach 40minütigem Rühren bei 0° C wird die Lösung in eine Mischung aus 5 Liter Eiswasser und 150 ml 2N Salzsäure gegossen. Die organische Phase wird mit kaltem Wasser gewaschen, über Magnesiumsulfat getrocknet und anschliessend eingedampft. Man erhält 379,3 g 2-Methylsulfonyloxy-3-nitrobenzaldehyd in Form eines Oeles. Zur weiteren Reinigung wird eine Portion von 42 g 2-Methylsulfonyloxy-3-nitrobenzaldehyd mit einer Mischung aus einem Teil Aethylacetat und 5 Teilen Hexan an Kieselgel chromatographiert. Man erhält 16,5 g gereinigtes 2-Methylsulfonyloxy-3-nitrobenzaldehyd, Smp. 98-99° C.

c) 2,2-Dioxo-8-nitro-1,2-benzoxathiin

Zu einer Lösung von 49 g 2-Methylsulfonyloxy-3-nitrobenzaldehyd in 450 ml Methylenchlorid und 112 ml Pyridin lässt man bei 0 bis +5° C innerhalb von 20 Minuten 32,8 ml 1,8-Diazabicyclo[5.4.0] undec-7-en zutropfen. Nach 15minütigem Rühren bei 0 bis +5° C erfolgt zweimal in Abständen von je 15 Minuten nacheinander tropfenweise Zugabe von Methylsulfonsäurechlorid und 1,8-Diazabicyclo[5.4.0] undec-7-en in folgenden Mengen:

1. 10,9 ml Methylsulfonsäurechlorid und 13,1 ml 1,8-Diazabicyclo[5.4.0] undec-7-en.
2. 5,5 ml Methylsulfonsäurechlorid und 13,1 ml 1,8-Diazabicyclo[5.4.0] undec-7-en.

Nach 30 Minuten werden bei 0° C 22,8 ml Phosphoroxychlorid innerhalb von 10 Minuten tropfenweise zugesetzt und es wird anschliessend für 30 Minuten gerührt.

Nach Zutropfen von 23,25 ml konzentrierter Schwefelsäure bei 0° C wird die Reaktionsmischung 3 Stunden bei dieser Temperatur gerührt. Der entstandene Niederschlag wird abfiltriert und der Filterrückstand mit Methylenchlorid gewaschen. Die vereinigten Filtrate werden mit 5 g Aktivkohle und 25 g Kieselgel entfärbt. Nach Filtrieren und Eindampfen der Lösung erhält man eine Rückstand in Form eines Oeles, der in 90 ml Methanol gelöst wird. Durch langsame Zugabe von 750 ml Wasser unter Kühlung wird das Produkt ausgefällt. Der Niederschlag wird abfiltriert und mit 250 ml Wasser gewaschen. Nach dem Trocknen erhält man 22 g 2,2-Dioxo-8-nitro-1,2-benzoxathiin, welches nach umkristallisieren aus einem Methanol/Wasser-Gemisch einen Schmelzpunkt von 127-128° C aufweist.

d) 8-Amino-2,2-dioxo-1,2-benzoxathiin

Zu einer siedenden Suspension aus 19 ml Eisessig, 7 ml Wasser, 5 ml Tetrahydrofuran und 5,5 g Eisenpulver lässt man innerhalb von 15 Minuten eine Lösung aus 1,5 g 2,2-Dioxo-8-nitro-1,2-benzoxathiin in 8 ml Tetrahydrofuran und 6 ml Eisessig zutropfen. Nach 15minütigem Rühren unter Rückfluss wird abfiltriert und das Filtrat eingedampft. Der Rückstand wird mit einer Mischung aus Eiswasser und Aethylacetat extrahiert. Nach Trocknen und Eindampfen der organischen Phase erhält man 1,3 g 8-Amino-2,2-dioxo-1,2-benzoxathiin, Smp. 79-81° C.

e) Eine Lösung aus 1,99 g 8-Amino-2,2-dioxo-1,2-benzoxathiin und 2,47 g 5,7-Dimethyl-1,2,4-triazolo[1,5-a] pyrimidin-2-yl-sulfonylchlorid in 16 ml getrocknetem Pyridin wird für eine Stunde bei Raumtemperatur gerührt. Anschliessend wird das Reaktionsgemisch eingedampft und der Rückstand zwischen 80 ml halbgesättigter Natriumcarbonatlösung und 40 ml Aethylacetat verteilt. Die Lösung wird filtriert, der Filterrückstand mit Aethylacetat gewaschen und anschliessend in Wasser suspendiert. Die Suspension wird mit 2N Salzsäure auf einen pH-Wert von pH 2 eingestellt. Nach Abfiltrieren und Trocknen des Rückstandes erhält man 3,7 g N-(2,2-Dioxo-1,2-benzoxathiin-8-yl)-5, 7-dimethyl-1,2,4-triazolo[ 1,5-a] pyrimidin-2-yl-sulfonamid, Smp. 214-215° C.

<u>Beispiel H2: N-(3,4-Dihydro-2,2-dioxo-3-methyl-1,2-benzoxathiin-8-yl)-5,7-dimethyl-1,2,4-triazolo[1,5-a] pyrimidin-2-yl-sulfonamid (Verbindung 1.02)</u>

a) 6-Brom-2,2-dioxo-3-methyl-8-nitro-1,2-benzoxathiin

Zu einer Lösung aus 49,2 g 5-Brom-2-hydroxy-3-nitrobenzaldehyd in 300 ml getrocknetem Methylenchlorid und 112,7 ml Pyridin lässt man bei 0 bis +5° C innerhalb von 15 Minuten 22,74 ml Aethylsulfonylchlorid zutropfen. Nach 15 Minuten erfolgt tropfenweise Zugabe von 65,52 ml 1,8-Diazabicyclo[5.4.0] undec-7-en. Nach 15 Minuten werden bei 0 bis +5° C innerhalb von 15 Minuten 7,58 ml Aethylsulfonylchlorid und 6 ml 1,8 Diazabicyclo[5.4.0] undec-7-en zugegeben. Nach 20 Minuten werden bei 0 bis +5° C innerhalb von 20 Minuten 22,8 ml Phosphoroxychlorid tropfenweise zugesetzt. Nach 90 Minuten erfolgt bei 0 bis +10° C innerhalb von 25 Minuten tropfenweise Zugabe von 22,52 ml konzentrierter Schwefelsaure. Die Reaktionsmischung wird für 18 Stunden bei 0° C gehalten und anschliessend filtriert. Der Filterrückstand wird mit Methylenchlorid gewaschen. Die vereinigten Filtrate werden mit Wasser extrahiert und anschliessend über Magnesiumsulfat getrocknet. Nach Behandlung der Lösung mit 5 g Aktivkohle wird über 800 g Kieselgel filtriert und das erhaltene Filtrat eingedampft. Der Rückstand wird durch Verreiben mit 120 ml Methanol kristallisiert. Man erhält 33,3 g 6-Brom-2,2-dioxo-3-methyl-8-nitro-1,2-benzoxathiin, Smp. 154-156° C.

b) 8-Amino-3,4-dihydro-2,2-dioxo-3-methyl-1,2-benzoxathiin

Eine Lösung aus 94,5 g 6-Brom-2,2-dioxo-3-methyl-8-nitro-1,2-benzoxathiin in 950 ml Tetrahydrofuran wird unter Zusatz von 29,5 g Magnesiumoxid und einem Palladium auf Aktivkohle-Katalysator (10 %) bei +40 bis +45° C und einem Druck von 20 bar 17 Stunden lang mit Wasserstoff hydriert. Anschliessend wird abfiltriert, das Filtrat eingedampft und der entstehende Rückstand in 500 ml Chloroform gelöst. Nach Zugabe von 250 ml Petroläther wird der entstehende Magnesiumdibromid-Niederschlag abfiltriert und das Filtrat eingedampft. Der Rückstand wird durch Verreiben mit wenig Aether kristallisiert. Man erhält 51,6 g 8-Amino-3,4-dihydro-2,2-dioxo-3-methyl-1,2-benzoxathiin, Smp. 97-98° C.

c) Eine Mischung aus 0,454 g 8-Amino-3,4-dihydro-2,2-dioxo-3-methyl-1,2-benzoxathiin und 0,5 g 5,7-Dimethyl-1,2,4-triazolo[1,5-a]pyrimidin-2-ylsulfonylchlorid wird 2 Stunden bei Raumtemperatur gerührt. Das Reaktionsgemisch wird eingedampft und zweimal mit halbgesättigter Natriumcarbonat-Lösung und Aethylacetat extrahiert. Die vereinigten wässrigen Phasen werden durch Zugabe von 2N Salzsäure bis auf einen pH-Wert von pH 2 angesäuert und zweimal mit Methylenchlorid extrahiert. Anschliessend werden die organischen Phasen vereinigt und nach dem Trocknen eingedampft. Man erhält 0,5 g N-(3,4-Dihydro-2,2,-dioxo-3-methyl-1,2-benzoxathiin-8-yl)-5,7-dimethyl-1,2,4-triazolo[1,5-a]pyrimidin-2-yl-sulfonamid, Smp. 229-231° C.

In analoger Weise werden die in den angeschlossenen Tabellen aufgelisteten Verbindungen der Formel I sowie die Zwischenprodukte der Formeln II und V hergestellt.

Tabelle 1

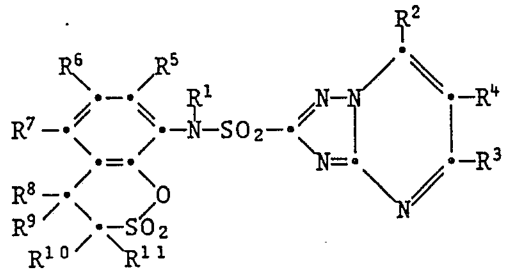

| Nr. | $R^1$ | $R^2$ | $R^3$ | $R^4$ | $R^5$ | $R^6$ | $R^7$ | $R^8$ | $R^9$ | $R^{10}$ | $R^{11}$ | Smp. [°C] |
|---|---|---|---|---|---|---|---|---|---|---|---|---|
| 1.01 | H | $CH_3$ | $CH_3$ | H | H | H | H | H | H | H | H | 214-215 |
| 1.02 | H | $CH_3$ | $CH_3$ | H | H | H | H | H | H | H | $CH_3$ | 229-231 |
| 1.03 | $CH_3$ | $CH_3$ | $CH_3$ | H | H | H | H | H | H | H | $CH_3$ | |
| 1.04 | H | $CH_3$ | $CH_3$ | H | H | H | H | H | H | $CH_3$ | $CH_3$ | |
| 1.05 | H | $CH_3$ | $CH_3$ | H | H | H | H | $CH_3$ | H | H | H | 233-234 |
| 1.06 | H | $CH_3$ | $CH_3$ | H | H | H | H | $CH_3$ | H | H | $CH_3$ | |
| 1.07 | H | $CH_3$ | $CH_3$ | H | H | H | H | H | H | $C_2H_5$ | $CH_3$ | |
| 1.08 | H | $CH_3$ | $CH_3$ | H | H | H | H | H | H | $C_3H_7$-i | $CH_3$ | |
| 1.09 | H | $CH_3$ | $CH_3$ | H | Cl | H | H | H | H | H | H | |
| 1.10 | H | $CH_3$ | $CH_3$ | H | Cl | H | H | H | H | H | $CH_3$ | |
| 1.11 | H | $CH_3$ | $CH_3$ | H | $COOCH_3$ | H | H | H | H | H | $CH_3$ | |
| 1.12 | H | H | $CH_3$ | H | H | H | H | H | H | H | $CH_3$ | |
| 1.13 | H | H | $CH_3$ | H | Cl | H | H | H | H | H | $CH_3$ | |
| 1.14 | H | H | $CH_3$ | H | $COOCH_3$ | H | H | H | H | H | H | |
| 1.15 | H | $CH_3$ | H | H | H | H | H | H | H | H | $CH_3$ | 242-243 |
| 1.16 | H | H | H | $CH_3$ | H | H | H | H | H | H | $CH_3$ | |
| 1.17 | H | H | $OCH_3$ | H | H | H | H | H | H | H | $CH_3$ | |
| 1.18 | H | $CH_3$ | $CH_3$ | $CH_3$ | H | H | H | H | H | H | $CH_3$ | |
| 1.19 | H | $CH_3$ | $OCH_3$ | H | H | H | H | H | H | H | $CH_3$ | |
| 1.20 | H | $CH_3$ | $OCH_3$ | H | $CH_3$ | H | H | H | H | H | $CH_3$ | |
| 1.21 | H | $CH_3$ | $OCH_3$ | H | $CH_3$ | H | H | H | H | H | H | |
| 1.22 | H | H | $CH_3$ | $CH_3$ | H | H | H | H | H | H | $CH_3$ | |
| 1.23 | H | $CH_3$ | $CH_3$ | Cl | H | H | H | H | H | H | $CH_3$ | |
| 1.24 | H | H | $OC_2H_5$ | H | H | H | H | H | H | H | $CH_3$ | |
| 1.25 | H | $CH_3$ | $CH_3$ | H | H | H | H | $C_2H_5$ | H | H | H | |
| 1.26 | H | $CH_3$ | $CH_3$ | H | H | H | H | H | H | H | $C_2H_5$ | |
| 1.27 | H | $CH_3$ | $CH_3$ | H | H | H | H | H | H | H | $C_3H_7$-i | |
| 1.28 | H | $CH_3$ | $CH_3$ | H | $CH_3$ | H | H | H | H | H | $CH_3$ | |
| 1.29 | H | $CH_3$ | $CH_3$ | H | $CON(CH_3)_2$ | H | H | H | H | H | $CH_3$ | |

Tabelle 2

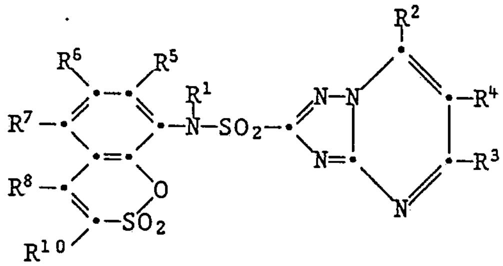

| Nr. | $R^1$ | $R^2$ | $R^3$ | $R^4$ | $R^5$ | $R^6$ | $R^7$ | $R^8$ | $R^{10}$ | Smp. [°C] |
|---|---|---|---|---|---|---|---|---|---|---|
| 2.01 | H | $CH_3$ | $CH_3$ | H | H | H | H | H | $CH_3$ | > 250 |
| 2.02 | $CH_3$ | $CH_3$ | $CH_3$ | H | H | H | H | H | $CH_3$ | |
| 2.03 | H | H | $OCH_3$ | H | H | H | H | H | $CH_3$ | |
| 2.04 | H | H | $CH_3$ | $CH_3$ | H | H | H | H | $CH_3$ | |
| 2.05 | H | $CH_3$ | $CH_3$ | H | H | H | H | H | H | > 250 |
| 2.06 | H | $CH_3$ | $CH_3$ | H | H | H | H | $C_2H_5$ | H | |
| 2.07 | H | $CH_3$ | $CH_3$ | H | H | H | H | H | $C_2H_5$ | |
| 2.08 | H | $CH_3$ | $CH_3$ | H | H | H | H | $CH_3$ | $CH_3$ | |
| 2.09 | H | $CH_3$ | $CH_3$ | H | H | H | H | $CH_3$ | H | 240-242 |
| 2.10 | H | $CH_3$ | $CH_3$ | H | H | Br | H | H | $CH_3$ | 228-230 |
| 2.11 | H | $CH_3$ | $CH_3$ | H | H | Br | H | H | H | |
| 2.12 | H | $CH_3$ | $CH_3$ | H | H | H | H | H | $C_3H_7$-i | |

Tabelle 3

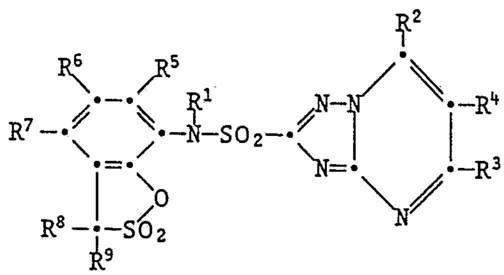

| Nr. | $R^1$ | $R^2$ | $R^3$ | $R^4$ | $R^5$ | $R^6$ | $R^7$ | $R^8$ | $R^9$ | Smp. [°C] |
|---|---|---|---|---|---|---|---|---|---|---|
| 3.01 | H | $CH_3$ | $CH_3$ | H | H | H | H | H | H | |
| 3.02 | H | $CH_3$ | $CH_3$ | H | H | H | H | $CH_3$ | $CH_3$ | |
| 3.03 | H | $CH_3$ | $CH_3$ | H | H | H | H | $CH_3$ | H | |
| 3.04 | H | $CH_3$ | $CH_3$ | H | H | H | H | H | $CH_3$ | |

Tabelle 4

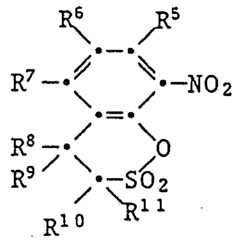

| Nr. | $R^5$ | $R^6$ | $R^7$ | $R^8$ | $R^9$ | $R^{10}$ | $R^{11}$ | Smp. [°C] |
|---|---|---|---|---|---|---|---|---|
| 4.01 | $COOCH_3$ | H | H | H | H | H | H | |
| 4.02 | H | H | H | $CH_3$ | H | H | $CH_3$ | |

Tabelle 5

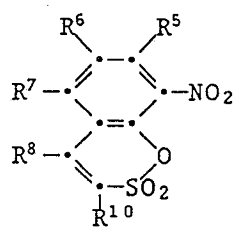

| Nr. | $R^5$ | $R^6$ | $R^7$ | $R^8$ | $R^{10}$ | Smp. [°C] |
|---|---|---|---|---|---|---|
| 5.01 | H | H | H | H | $CH_3$ | 91-96 |
| 5.02 | H | H | H | H | $C_2H_5$ | 140-143 |
| 5.03 | H | H | H | H | H | 127-128 |
| 5.04 | Cl | H | H | H | H | |
| 5.05 | Cl | H | H | H | $CH_3$ | |
| 5.06 | $COOCH_3$ | H | H | H | $CH_3$ | |
| 5.07 | $COOCH_3$ | H | H | H | H | |
| 5.08 | H | Br | H | H | $CH_3$ | 154-156 |
| 5.09 | H | H | H | $CH_3$ | $CH_3$ | |
| 5.10 | H | Br | H | $CH_3$ | H | 141-142 |
| 5.11 | H | Br | H | $CH_3$ | $CH_3$ | 186-188 |
| 5.12 | H | Br | H | H | $C_3H_7$-n | 119-121 |

Tabelle 6

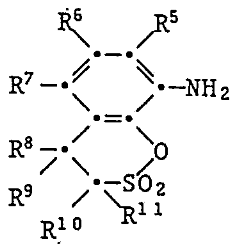

| Nr. | $R^5$ | $R^6$ | $R^7$ | $R^8$ | $R^9$ | $R^{10}$ | $R^{11}$ | Smp. [°C] |
|---|---|---|---|---|---|---|---|---|
| 6.01 | H | H | H | H | H | H | $CH_3$ | 97-98 |
| 6.02 | H | H | H | H | H | H | $C_2H_5$ | Oel |
| 6.03 | H | H | H | H | H | H | H | 112-113 |
| 6.04 | H | H | H | H | H | H | $C_3H_7$-n | 61-62 |
| 6.05 | Cl | H | H | H | H | H | H | |
| 6.06 | Cl | H | H | H | H | H | $CH_3$ | |
| 6.07 | $COOCH_3$ | H | H | H | H | H | H | |
| 6.08 | $COOCH_3$ | H | H | H | H | H | $CH_3$ | |
| 6.09 | H | H | H | $CH_3$ | H | H | $CH_3$ | |
| 6.10 | H | H | H | $CH_3$ | H | H | H | 79-81 |
| 6.11 | H | Br | H | H | H | H | $CH_3$ | |

Tabelle 7

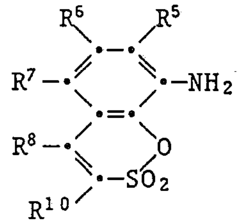

| Nr. | $R^5$ | $R^6$ | $R^7$ | $R^8$ | $R^{10}$ | Smp. [°C] |
|---|---|---|---|---|---|---|
| 7.01 | H | H | H | H | $CH_3$ | |
| 7.02 | H | H | H | H | $C_2H_5$ | |
| 7.03 | H | H | H | H | H | 79-81 |
| 7.04 | H | Br | H | H | $C_2H_5$ | 114-116 |
| 7.05 | H | Br | H | H | H | 148-149 |
| 7.06 | $COOCH_3$ | H | H | H | $CH_3$ | |
| 7.07 | H | H | H | $CH_3$ | H | 182-184 |
| 7.08 | H | Br | H | $CH_3$ | H | 164-165 |
| 7.09 | H | Br | H | $CH_3$ | $CH_3$ | 157-159 |
| 7.10 | H | H | H | $CH_3$ | $CH_3$ | |

Tabelle 8

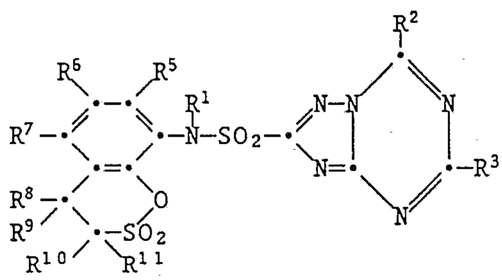

| Nr. | $R^1$ | $R^2$ | $R^3$ | $R^5$ | $R^6$ | $R^7$ | $R^8$ | $R^9$ | $R^{10}$ | $R^{11}$ | Smp. [°C] |
|---|---|---|---|---|---|---|---|---|---|---|---|
| 8.01 | H | $CH_3$ | $CH_3$ | H | H | H | H | H | H | $CH_3$ | |
| 8.02 | H | $CH_3$ | $CH_3$ | Cl | H | H | H | H | H | $CH_3$ | |
| 8.03 | H | $CH_3$ | $CH_3$ | Br | H | H | H | H | H | $CH_3$ | |
| 8.04 | H | $CH_3$ | $CH_3$ | $COOCH_3$ | H | H | H | H | H | $CH_3$ | |
| 8.05 | H | $OCH_3$ | $CH_3$ | H | H | H | H | H | H | $CH_3$ | |
| 8.06 | H | $OCH_3$ | $CH_3$ | H | H | H | H | H | H | H | |
| 8.07 | H | $CH_3$ | $OCH_3$ | H | H | H | H | H | H | $CH_3$ | |
| 8.08 | H | $CH_3$ | $OCH_3$ | H | H | H | H | H | H | H | |

Tabelle 9

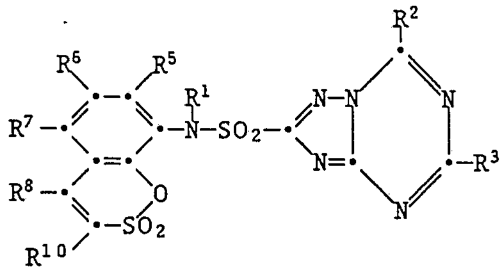

| Nr. | $R^1$ | $R^2$ | $R^3$ | $R^5$ | $R^6$ | $R^7$ | $R^8$ | $R^{10}$ | Smp. [°C] |
|---|---|---|---|---|---|---|---|---|---|
| 9.01 | H | $CH_3$ | $CH_3$ | H | H | H | H | H | |
| 9.02 | H | $CH_3$ | $CH_3$ | H | H | H | H | $CH_3$ | |
| 9.03 | H | $CH_3$ | $CH_3$ | Br | H | H | H | $CH_3$ | |
| 9.04 | H | $CH_3$ | $CH_3$ | $COOCH_3$ | H | H | H | $CH_3$ | |
| 9.05 | H | $OCH_3$ | $CH_3$ | H | H | H | H | $CH_3$ | |
| 9.06 | H | $OCH_3$ | $CH_3$ | H | H | H | H | H | |
| 9.07 | H | $CH_3$ | $OCH_3$ | H | H | H | H | H | |
| 9.08 | H | $CH_3$ | $OCH_3$ | H | H | H | H | $CH_3$ | |
| 9.09 | H | $OCH_3$ | $CH_3$ | $COOCH_3$ | H | H | H | $CH_3$ | |
| 9.10 | H | $CH_3$ | $OCH_3$ | $COOCH_3$ | H | H | H | $CH_3$ | |

Formulierungsbeispiele:

Beispiel F 1: Formulierungsbeispiele für Wirkstoffe der Formel I (% = Gewichtsprozent)

| a) Spritzpulver | a) | b) | c) |
|---|---|---|---|
| Wirkstoff Nr. 2.05 | 20 % | 50 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther /7-8 Mol AeO) | - | 2 % | 2 % |
| Hochdisperse Kieselsäure | 5 % | 27 % | 27 % |
| Kaolin | 67 % | 10 % | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff Nr. 2.05 | 10 % | 1 % |
| Octylphenolpolyäthylenglykoläther (4-5 Mol AeO) | 3 % | 3 % |
| Ca-Dodecylbenzosulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff Nr. 1.02 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff Nr. 2.05 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff Nr. 2.05 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff Nr. 1.02 | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff Nr. 1.02 | 5 % |
| Isopropylamin | 1 % |
| Octylphenolpolyäthylenglykoläther (78 Mol AeO) | 3 % |
| Wasser | 91 % |

Biologische Beispiele:

Beispiel B 1: Herbizidwirkung vor dem Auflaufen der Pflanzen

Kunststofftöpfe werden mit expandiertem Vermiculit (Dichte: 0,135 g/$cm^3$, Wasserabsorptionsvermögen: 0,565 1/1) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 klux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5 % eines handelsüblichen Flüssigdüngers (®Greenzit, ex Ciba-Geigy) zugesetzt. 12 Tage nach der Aussaat wird der Versuch ausgewertet und die Wirkung auf die Versuchspflanzen nach dem folgenden Massstab bewertet:

1 : Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

In diesem Versuch zeigten die Verbindungen der Tabellen 1 bis 3 eine starke Herbizidwirkung.

Beispiel B2: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat behandelt. Es werden Konzentrationen von 0,5 kg Wirksubstanz/Hektar angewendet. Die Saatschalen werden im Gewächshaus bei 22-25°C und 50-70 relativer Luftfeuchtigkeit gehalten und der Versuch nach 3 Wochen nach dem im Beispiel B1 angegebenen Massstab ausgewertet.

Pre-emergente Wirkung
Konzentration: 0,5 kg Wirkstoff pro Hektar

| Testpflanze<br>Wirkstoff Nr. | Avena | Setaria | Sinapis | Stellaria |
|---|---|---|---|---|
| 1.02 | 2 | 1 | 2 | 2 |

Beispiel B3: Post-emergente Herbizid-Wirkung (Kontaktherbizid)

Eine Anzahl Unkräuter, sowohl monokytle wie dikotyle, wurden nach dem Auflaufen (im 4- bis 6-Blattstadium) mit einer wässrigen Wirkstoffdispersion in einer Dosierung von 0,5 kg Wirksubstanz pro Hektar behandelt diese bei 24-26°C und 45-60 % relativer Luftfeuchtigkeit gehalten. 15 Tage nach der Behandlung wird der Versuch nach dem im Beispiel B1 angegbenen Massstab ausgewertet.

Post-emergente Wirkung
Konzentration: 0,5 kg Wirkstoff pro Hektar

| Testpflanze<br>Wirkstoff Nr. | Avena | Setaria | Lolium | Sinapis | Solanum | Stellaria | Phaseolus |
|---|---|---|---|---|---|---|---|
| 1.02 | 4 | 4 | 3 | 2 | 4 | 2 | 2 |

**Patentansprüche**

1. N-Benzoxathiolanyl- und N-Benzoxathiinyl-triazolopyrimidinyl- und -triazolotriazinylsulfonamide der Formel I

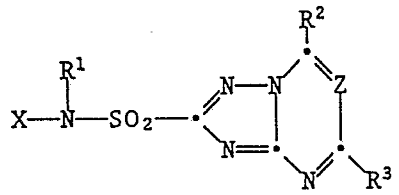

(I)

worin X einen Rest der Formel

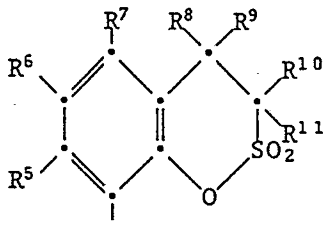

X1

,

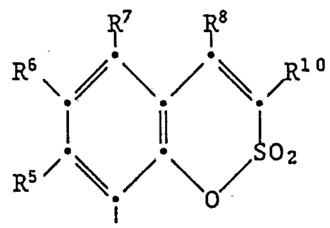

X2

oder

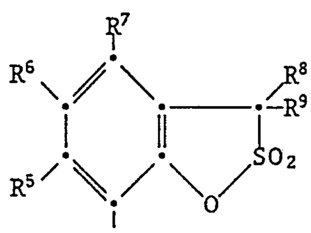

X3

,

Z Stickstoff oder $CR^4R^1$ Wasserstoff, $C_1$-$C_6$-Alkyl, durch Halogen, $C_1$-$C_4$-Alkoxycarbonyl, $C_2$-$C_6$-Alkenyl oder $C_2$-$C_6$-Alkinyl substituiertes $C_1$-$C_6$-Alkyl, Benzyl oder durch Halogen, Methyl, Methoxy, -CO-$R^{12}$, -CO-$OR^{12}$, -$SO_2$-$R^{12}$ oder -CO-$NR^{13}R^{14}$ substituiertes Benzyl,

$R^2$, $R^3$ und $R^4$ unabhänig voneinander Wasserstoff, $C_1$-$C_6$-Alkyl, $C_3$-$C_6$-Cycloalkyl, $C_1$-$C_6$-Halogenalkyl, $C_1$-$C_6$-Alkoxy, oder

$R^2$ und $R^4$ zusammen oder $R^3$ und $R^4$ zusammen eine $C_3$ bis $C_4$-Alkylenkette oder eine $C_2$-$C_4$-Alkylenkette, worin ein Kettenglied durch Sauerstoff, Schwefel, -SO-, -$SO_2$-, -NH- oder -N($C_1$-$C_4$-Alkyl)- ersetzt werden kann,

$R^5$, $R^6$ und $R^7$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkoxycarbonyl, $C_2$-$C_4$-Alkoxyalkoxy oder -CO-$NR^{15}R^{16}$, mit der Massgabe, dass stets einer der Reste $R^5$, $R^6$ und $R^7$ für Wasserstoff steht,

$R^8$, $R^9$, $R^{10}$ und $R^{11}$ unabhängig voneinander Wasserstoff oder $C_1$-$C_4$-Alkyl, oder

$R^8$ und $R^9$ zusammen oder $R^{10}$ und $R^{11}$ zusammen mit dem sie tragenden Kohlenstoffatom einen spirocyclisch verknüpften $C_3$ bis $C_6$-Cycloalkylring bilden,

$R^{12}$ $C_1$-$C_4$-Alkyl, Phenyl, Benzyl, durch Halogen, Methyl oder Trifluoromethyl substituiertes Phenyl oder durch Halogen, Methyl oder Trifluormethyl substituiertes Benzyl, und

$R^{13}$, $R^{14}$, $R^{15}$ und $R^{16}$ unabhängig voneinander Wasserstoff oder $C_1$-$C_4$-Alkyl bedeuten.

2. Verbindungen gemäss Anspruch 1, dadurch gekennzeichnet, dass X die Gruppe X1 bedeutet.

3. Verbindungen gemäss Anspruch 1, dadurch gekennzeichnet, dass X die Gruppe X2 bedeutet.

4. Verbindungen gemäss Anspruch 2, dadurch gekennzeichnet, dass X die Gruppe X1 bedeutet, wobei zwei von $R^5$, $R^6$ und $R^7$ unabhängig voneinander für Wasserstoff, Halogen, $C_1$-$C_4$-Alkyl oder $C_1$-$C_4$-Alkoxy stehen und der dritte Wasserstoff bedeutet.

5. Verbindungen gemäss Anspruch 4, dadurch gekennzeichnet, dass zwei von $R^5$, $R^6$ und $R^7$ unabhängig voneinander für Wasserstoff, Methyl, Chlor oder Methoxy stehen und der dritte Wasserstoff bedeutet.

6. Verbindungen gemäss Anspruch 3, dadurch gekennzeichnet, dass X die Gruppe X2 bedeutet, wobei zwei von $R^5$, $R^6$ und $R^7$ unabhängig voneinander für Wasserstoff, Halogen, $C_1$-$C_4$-Alkyl oder $C_1$-$C_4$-Alkoxy stehen und der dritte Wasserstoff bedeutet.

7. Verbindungen gemäss Anspruch 6, dadurch gekennzeichnet, dass zwei von $R^5$, $R^6$ und $R^7$ unabhängig voneinander für Wasserstoff, Methyl, Chlor oder Methoxy stehen und der dritte Wasserstoff bedeutet.

8. Verbindungen gemäss Anspruch 4, dadurch gekennzeichnet, dass $R^8$, $R^9$, $R^{10}$ und $R^{11}$ unabhängig

voneinander Wasserstoff oder Methyl bedeuten.

9. Verbindungen gemäss Anspruch 6, dadurch gekennzeichnet, dass $R^8$ und $R^{10}$ unabhängig voneinander Wasserstoff oder Methyl bedeuten.

10. Verbindungen gemäss Anspruch 1, dadurch gekennzeichnet, dass $R^1$ Wasserstoff bedeutet.

11. Verbindungen gemäss Anspruch 1, dadurch gekennzeichnet, dass 2 für die Gruppe $CR^4$ steht, wobei $R^4$ Wasserstoff oder Methyl bedeutet.

12. Verbindungen gemäss Anspruch 1, dadurch gekennzeichnet, dass $R^2$ und $R^3$ unabhängig voneinander Wasserstoff, Methyl oder Methoxy bedeuten.

13. Verbindungen gemäss Anspruch 1, dadurch gekennzeichnet, dass $R^1$ Wasserstoff, $R^2$ und $R^3$ unabhängig voneinander Wasserstoff, Methyl oder Methoxy und Z die Gruppe CH oder $C(CH_3)$ bedeuten.

14. Verbindungen gemäss Anspruch 1, dadurch gekennzeichnet, dass X die Gruppe X1, $R^1$ Wasserstoff, $R^2$ und $R^3$ unabhängig voneinander Wasserstoff, Methyl oder Methoxy, zwei von $R^5$, $R^6$ und $R^7$ unabhängig voneinander Wasserstoff, Methyl, Chlor oder Methoxy, der dritte von $R^5$, $R^6$ und $R^7$ Wasserstoff, und $R^8$, $R^9$, $R^{10}$ und $R^{11}$ unabhängig voneinander Wasserstoff oder Methyl bedeuten.

15. Verbindungen gemäss Anspruch 14, dadurch gekennzeichnet, dass Z die Gruppe CH oder $C(CH_3)$ bedeutet.

16. Verbindungen gemäss Anspruch 1, dadurch gekennzeichnet, dass X die Gruppe X2, $R^1$ Wasserstoff, $R^2$ und $R^3$ unabhängig voneinander Wasserstoff, Methyl oder Methoxy, zwei von $R^5$, $R^6$ und $R^7$ unabhängig voneinander Wasserstoff, Methyl, Chlor oder Methoxy, der dritte von $R^5$, $R^6$ und $R^7$ Wasserstoff, und $R^8$ und $R^{10}$ unabhängig voneinander Wasserstoff oder Methyl bedeuten.

17. Verbindungen gemäss Anspruch 16, dadurch gekennzeichnet, dass Z die Gruppe CH oder $C(CH_3)$ bedeutet.

18. N-(2,2-Dioxo-1,2-benzoxathiin-8-yl)-5,7-dimethyl-1,2,4-triazolo [1,5-a] pyrimidin-2-yl-sulfonamid gemäss Anspruch 1.

19. N-(3,4-Dihydro-2,2-dioxo-3-methyl-1,2-benzoxathiin-8-yl)-5,7-dimethyl-1,2,4-triazolo[1,5-a] pyrimidin-2-yl-sulfonamid gemäss Anspruch 1.

20. Verfahren zur Herstellung der Verbindungen der Formel I gemäss Anspruch 1, dadurch gekennzeichnet, dass man ein primäres Amin der Formel II

$X-NH_2$ (II)

worin X die unter Formel I gegebene Bedeutung hat, in Gegenwart einer Base mit einem Triazolopyrimidinyl- oder Triazolotriazinylsulfonylchlorid der Formel III

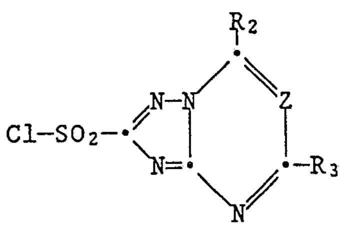

(III)

worin $R^2$, $R^3$ und Z die unter Formel I gegebenen Bedeutungen haben, umsetzt und gewünschtenfalls das erhaltene sekundäre Amin der Unterformel Ia

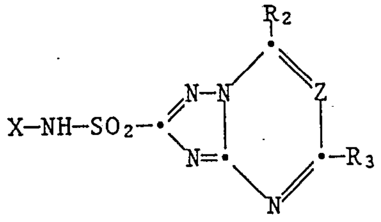

(Ia)

worin $R^2$, $R^3$, X und Z die unter Formel I gegebenen Bedeutungen haben, in Gegenwart einer Base mit einer Verbindung der Formel IV

$R^1-Y$ (IV)

worin $R^1$ mit Ausnahme von Wasserstoff die unter Formel I gegebene Bedeutung hat und Y für eine leaving group steht, alkyliert.

21. Ein herbizides Mittel, dadurch gekennzeichnet, dass es neben Träger-und/oder anderen Zuschlagstoffen als Wirkstoff mindestens ein Triazolylsulfonamid der Formel I, gemäss Anspruch 1, enthält.

22. Mittel gemäss Anspruch 21, dadurch gekennzeichnet, dass es zwischen 0,1 % und 95 % Wirkstoff der Formel I gemäss Anspruch 1 enthält.

23. Verfahren zur Bekämpfung unerwünschten Pflanzenwachstums, dadurch gekennzeichnet, dass man einen Wirkstoff der Formel I, gemäss Anspruch 1, oder ein diesen Wirkstoff enthaltendes Mittel in einer

wirksamen Menge auf die Pflanzen oder deren Lebensraum appliziert.

24. Verfahren gemäss Anspruch 23, dadurch gekennzeichnet, dass man eine Wirkstoffmenge zwischen 0,01 und 10 kg pro Hektar appliziert.

25. Verfahren gemäss Anspruch 23, zur selektiven pre- oder post-emergenten Bekämpfung von Unkräutern in Nutzpflanzenkulturen.

26. Verbindungen der Formel II

$X\text{-}NH_2$ (II)

worin

X einen Rest der Formel

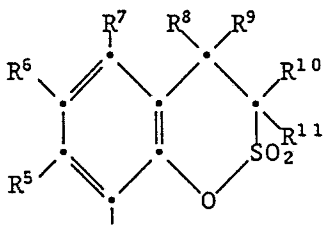

X1

,

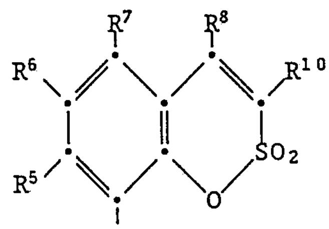

X2

oder

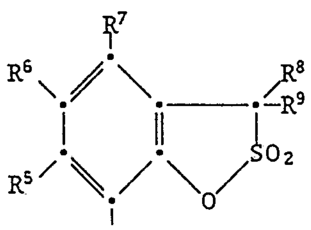

X3

,

$R^5$, $R^6$ und $R^7$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkoxycarbonyl, $C_2$-$C_4$-Alkoxyalkoxy oder $-CO-NR^{15}R^{16}$ mit der Massgabe, dass stets einer der Reste $R^5$, $R^6$ und $R^7$ für Wasserstoff steht,

$R^8$, $R^9$, $R^{10}$ und $R^{11}$ unabhängig voneinander Wasserstoff oder $C_1$-$C_4$-Alkyl, oder

$R^8$ und $R^9$ zusammen oder $R^{10}$ und $R^{11}$ zusammen mit dem sie tragenden Kohlenstoffatom einen spirocyclisch verknüpften $C_3$ bis $C_6$-Cycloalkylring bilden, und

$R^{15}$ und $R^{16}$ unabhängig voneinander Wasserstoff oder $C_1$-$C_4$-Alkyl bedeuten.

27. Verfahren zur Herstellung der Verbindungen der Formel II, gemäss Anspruch 26, dadurch gekennzeichnet, dass man eine Verbindung der Formel V

$X\text{-}NO_2$ (V)

worin X die unter Formel II im Anspruch 26 gegebene Bedeutung hat, mit einem Reduktionsmittel reduziert.

28. Verbindungen der Formel V

$X\text{-}NO_2$ (V)

worin

X einen Rest der Formel

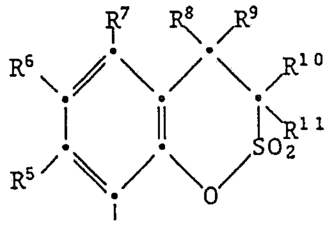

X1

,

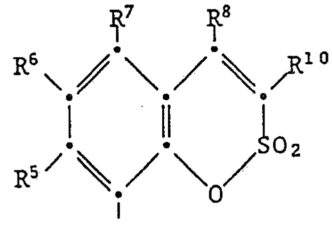

X2

oder

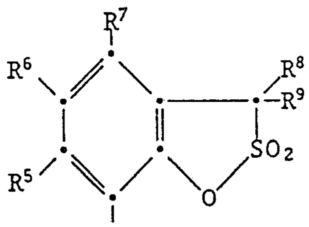

X3

,

$R^5$, $R^6$ und $R^7$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkoxycarbonyl, $C_2$-$C_4$-Alkoxyalkoxy oder -CO-$NR^{15}R^{16}$ mit der Massgabe, dass stets einer der Reste $R^5$, $R^6$ und $R^7$ für Wasserstoff steht,
$R^8$, $R^9$, $R^{10}$ und $R^{11}$ unabhängig voneinander Wasserstoff oder $C_1$-$C_4$-Alkyl, oder
$R^8$ und $R^9$ zusammen oder $R^{10}$ und $R^{11}$ zusammen mit dem sie tragenden Kohlenstoffatom einen spirocyclisch verknüpften $C_3$ bis $C_6$-Cycloalkylring bilden, und
$R^{15}$ und $R^{16}$ unabhängig voneinander Wasserstoff oder $C_1$-$C_4$-Alkyl bedeuten, mit Ausnahme der Verbindung 5-Brom-7-nitro-2,2-dioxo-benzoxathiolan.

29. Verfahren zur Herstellung der Verbindungen der Formel V gemäss Anspruch 28, dadurch gekennzeichnet, dass man eine Verbindung der Formel VI
X-H (VI)
worin X die unter Formel V im Anspruch 28 gegebene Bedeutung hat, mit einem Nitrierungsmittel nitriert.

**Patentansprüche für folgenden Vertragsstaat: ES**

1. Verfahren zur Herstellung der Verbindungen der Formel I

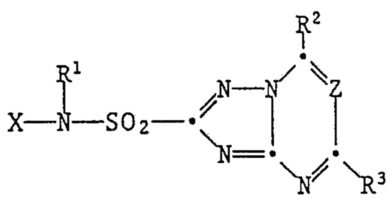

(I)

worin
X einen Rest der Formel

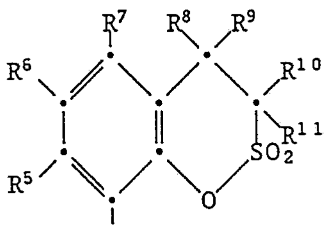

,

X1

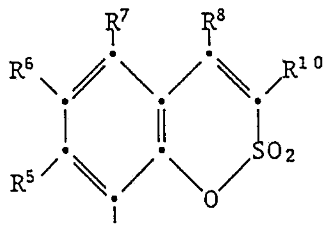

oder

X2

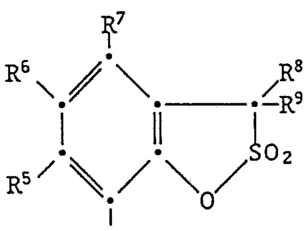

,

X3

Z Stickstoff oder $CR^4$,
$R^1$ Wasserstoff, $C_1$-$C_6$-Alkyl, durch Halogen, $C_1$-$C_4$-Alkoxycarbonyl, $C_2$-$C_6$-Alkenyl oder $C_2$-$C_6$-Alkinyl substituiertes $C_1$-$C_6$-Alkyl, Benzyl oder durch Halogen, Methyl, Methoxy, -CO-$R^{12}$, -CO-$OR^{12}$, -$SO_2$-$R^{12}$ oder -CO-$NR^{13}R^{14}$ substituiertes Benzyl,
$R^2$, $R^3$ und $R^4$ unabhänig voneinander Wasserstoff, $C_1$-$C_6$-Alkyl, $C_3$-$C_6$-Cycloalkyl, $C_1$-$C_6$-Halogenalkyl, $C_1$-$C_6$-Alkoxy, oder
$R^2$ und $R^4$ zusammen oder $R^3$ und $R^4$ zusammen eine $C_3$ bis $C_4$-Alkylenkette oder eine $C_2$-$C_4$-Alkylenkette, worin ein Kettenglied durch Sauerstoff, Schwefel, -SO-, -$SO_2$-, -NH- oder -N($C_1$-$C_4$-Alkyl)- ersetzt werden kann,
$R^5$, $R^6$ und $R^7$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkoxycarbonyl, $C_2$-$C_4$-Alkoxyalkoxy oder -CO-$NR^{15}R^{16}$, mit der Massgabe, dass stets einer der Reste $R^5$, $R^6$ und $R^7$ für Wasserstoff steht,
$R^8$, $R^9$, $R^{10}$ und $R^{11}$ unabhängig voneinander Wasserstoff oder $C_1$-$C_4$-Alkyl, oder
$R^8$ und $R^9$ zusammen oder $R^{10}$ und $R^{11}$ zusammen mit dem sie tragenden Kohlenstoffatom einen spirocyclisch verknüpften $C_3$ bis $C_6$-Cycloalkylring bilden,
$R^{12}$ $C_1$-$C_4$-Alkyl, Phenyl, Benzyl, durch Halogen, Methyl oder Trifluoromethyl substituiertes Phenyl oder durch Halogen, Methyl oder Trifluormethyl substituiertes Benzyl, und
$R^{13}$, $R^{14}$, $R^{15}$ und $R^{16}$ unabhängig voneinander Wasserstoff oder $C_1$-$C_4$-Alkyl
bedeuten, dadurch gekennzeichnet, dass man ein primäres Amin der Formel II
X-$NH_2$ (II)
worin X die unter Formel I gegebene Bedeutung hat, in Gegenwart einer Base mit einem Triazolopyrimidinyl- oder Triazolotriazinylsulfonylchlorid der Formel III

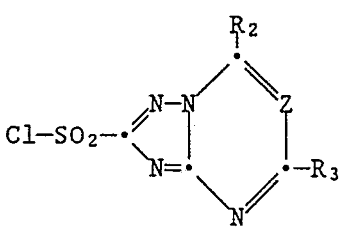

(III)

worin $R^2$, $R^3$ und Z die unter Formel I gegebenen Bedeutungen haben, umsetzt und gewünschtenfalls das erhaltene sekundäre Amin der Unterformel Ia

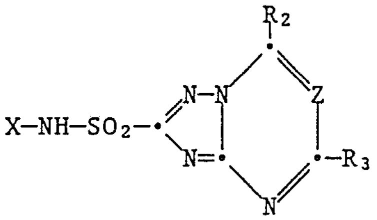

(Ia)

worin $R^2$, $R^3$, X und Z die unter Formel I gegebenen Bedeutungen haben, in Gegenwart einer Base mit einer Verbindung der Formel IV

$R^1$-Y (IV)

worin $R^1$ mit Ausnahme von Wasserstoff die unter Formel I gegebene Bedeutung hat und Y für eine leaving group steht, alkyliert.

2. Verfahren gemäss Anspruch 1, dadurch gekennzeichnet, dass X die Gruppe X1 bedeutet.

3. Verfahren gemäss Anspruch 1, dadurch gekennzeichnet, dass X die Gruppe X2 bedeutet.

4. Verfahren gemäss Anspruch 2, dadurch gekennzeichnet, dass X die Gruppe X1 bedeutet, wobei zwei von $R^5$, $R^6$ und $R^7$ unabhängig voneinander für Wasserstoff, Halogen, $C_1$-$C_4$-Alkyl oder $C_1$-$C_4$-Alkoxy stehen und der dritte Wasserstoff bedeutet.

5. Verfahren gemäss Anspruch 4, dadurch gekennzeichnet, dass zwei von $R^5$, $R^6$ und $R^7$ unabhängig voneinander für Wasserstoff, Methyl, Chlor oder Methoxy stehen und der dritte Wasserstoff bedeutet.

6. Verfahren gemäss Anspruch 3, dadurch gekennzeichnet, dass X die Gruppe X2 bedeutet, wobei zwei von $R^5$, $R^6$ und $R^7$ unabhängig voneinander für Wasserstoff, Halogen, $C_1$-$C_4$-Alkyl oder $C_1$-$C_4$-Alkoxy stehen und der dritte Wasserstoff bedeutet.

7. Verfahren gemäss Anspruch 6, dadurch gekennzeichnet, dass zwei von $R^5$, $R^6$ und $R^7$ unabhängig voneinander für Wasserstoff, Methyl, Chlor oder Methoxy stehen und der dritte Wasserstoff bedeutet.

8. Verfahren gemäss Anspruch 4, dadurch gekennzeichnet, dass $R^8$, $R^9$, $R^{10}$ und $R^{11}$ unabhängig voneinander Wasserstoff oder Methyl bedeuten.

9. Verfahren gemäss Anspruch 6, dadurch gekennzeichnet, dass $R^8$ und $R^{10}$ unabhängig voneinander Wasserstoff oder Methyl bedeuten.

10. Verfahren gemäss Anspruch 1, dadurch gekennzeichnet, dass $R^1$ Wasserstoff bedeutet.

11. Verfahren gemäss Anspruch 1, dadurch gekennzeichnet, dass 2 für die Gruppe $CR^4$ steht, wobei $R^4$ Wasserstoff oder Methyl bedeutet.

12. Verfahren gemäss Anspruch 1, dadurch gekennzeichnet, dass $R^2$ und $R^3$ unabhängig voneinander Wasserstoff, Methyl oder Methoxy bedeuten.

13. Verfahren gemäss Anspruch 1, dadurch gekennzeichnet, dass $R^1$ Wasserstoff, $R^2$ und $R^3$ unabhängig voneinander Wasserstoff, Methyl oder Methoxy und 2 die Gruppe CH oder $C(CH_3)$ bedeuten.

14. Verfahren gemäss Anspruch 1, dadurch gekennzeichnet, dass X die Gruppe X1, $R^1$ Wasserstoff, $R^2$ und $R^3$ unabhängig voneinander Wasserstoff, Methyl oder Methoxy, zwei von $R^5$, $R^6$ und $R^7$ unabhängig voneinander Wasserstoff, Methyl, Chlor oder Methoxy, der dritte von $R^5$, $R^6$ und $R^7$ Wasserstoff, und $R^8$, $R^9$, $R^{10}$ und $R^{11}$ unabhängig voneinander Wasserstoff oder Methyl bedeuten.

15. Verfahren gemäss Anspruch 14, dadurch gekennzeichnet, dass Z die Gruppe CH oder $C(CH_3)$ bedeutet.

16. Verfahren gemäss Anspruch 1, dadurch gekennzeichnet, dass X die Gruppe X2, $R^1$ Wasserstoff, $R^2$ und $R^3$ unabhängig voneinander Wasserstoff, Methyl oder Methoxy, zwei von $R^5$, $R^6$ und $R^7$ unabhängig voneinander Wasserstoff, Methyl, Chlor oder Methoxy, der dritte von $R^5$, $R^6$ und $R^7$ Wasserstoff, und $R^8$ und $R^{10}$ unabhängig voneinander Wasserstoff oder Methyl bedeuten.

17. Verfahren gemäss Anspruch 16, dadurch gekennzeichnet, dass Z die Gruppe CH oder $C(CH_3)$ bedeutet.

18. Verfahren gemäss Anspruch 1, dadurch gekennzeichnet, dass man N-(2,2-Dioxo-1,2-benzoxathiin-8-yl)-5,7-dimethyl-1,2,4-triazolo[1,5-a] pyrimidin-2-yl-sulfonamid gemäss Anspruch 1.

19. Verfahren gemäss Anspruch 1, dadurch gekennzeichnet, dass man N-(3,4-Dihydro-2,2-dioxo-3-methyl-1,2-benzoxathiin-8-yl)-5,7-dimethyl-1,2,4-triazolo[1,5-a] pyrimidin-2-yl-sulfonamid herstellt.

20. Verfahren zur Herstellung der Verbindungen der Formel II

$X$-$NH_2$ (II)

worin

X einen Rest der Formel

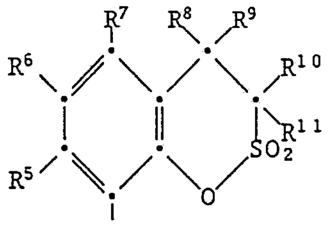

,

X1

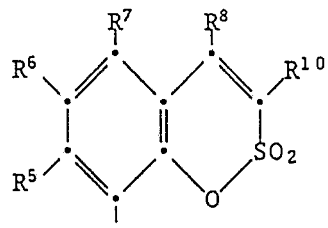

oder

X2

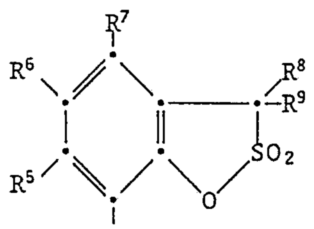

,

X3

$R^5$, $R^6$ und $R^7$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkoxycarbonyl, $C_2$-$C_4$-Alkoxyalkoxy oder -CO-$NR^{15}R^{16}$, mit der Massgabe, dass stets einer der Reste $R^5$, $R^6$ und $R^7$ für Wasserstoff steht,
$R^8$, $R^9$, $R^{10}$ und $R^{11}$ unabhängig voneinander Wasserstoff oder $C_1$-$C_4$-Alkyl, oder
$R^8$ und $R^9$ zusammen oder $R^{10}$ und $R^{11}$ zusammen mit dem sie tragenden Kohlenstoffatom einen spirocyclisch verknüpften $C_3$ bis $C_6$-Cycloalkylring bilden, und
$R^{15}$ und $R^{16}$ unabhängig voneinander Wasserstoff oder $C_1$-$C_4$-Alkyl bedeuten, dadurch gekennzeichnet, dass man eine Verbindung der Formel V
X-$NO_2$ (V)
worin X die unter Formel II im Anspruch 26 gegebene Bedeutung hat, mit einem Reduktionsmittel reduziert.

21. Verfahren zur Herstellung der Verbindungen der Formel V
X-$NO_2$ (V)
worin
X einen Rest der Formel

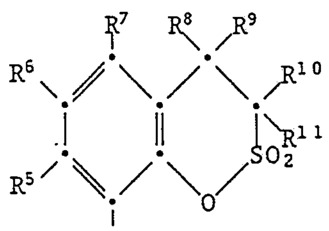

,

X1

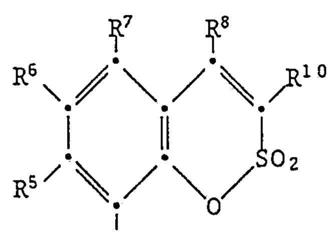

oder

X2

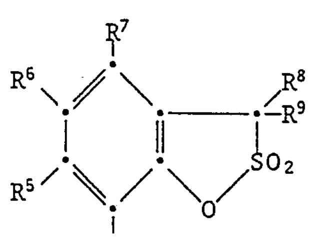

,

X3

$R^5$, $R^6$ und $R^7$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkoxycarbonyl, $C_2$-$C_4$-Alkoxyalkoxy oder -CO-$NR^{15}R^{16}$, mit der Massgabe, dass stets einer der Reste $R^5$, $R^6$ und $R^7$ für Wasserstoff steht,

$R^8$, $R^9$, $R^{10}$ und $R^{11}$ unabhängig voneinander Wasserstoff oder $C_1$-$C_4$-Alkyl, oder
$R^8$ und $R^9$ zusammen oder $R^{10}$ und $R^{11}$ zusammen mit dem sie tragenden Kohlenstoffatom einen spirocyclisch verknüpften $C_3$ bis $C_6$-Cycloalkylring bilden, und
$R^{15}$ und $R^{16}$ unabhängig voneinander Wasserstoff oder $C_1$-$C_4$-Alkyl bedeuten, mit Ausnahme der Verbindung 5-Brom-7-nitro-2,2-dioxo-benzoxathiolan,
dadurch gekennzeichnet, dass man eine Verbindung der Formel VI
X-H (VI)
worin X die unter Formel V im Anspruch 28 gegebene Bedeutung hat, mit einem Nitrierungsmittel nitriert.

Europäisches Patentamt

# EUROPÄISCHER RECHERCHENBERICHT

Nummer der Anmeldung

EP 89810255.3

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A2/A3 - 0 142 152 (THE DOW CHEMICAL COMPANY)<br>* Ansprüche 1,2,43,44,67 *<br>-- | 1,10-13,20-25 | C 07 D 487/04<br>C 07 D 327/04<br>C 07 D 327/06<br>A 01 N 43/90//<br>(C 07 D 487/04<br>C 07 D 249:00<br>C 07 D 239:00)<br>(C 07 D 487/04<br>C 07 D 251:00<br>C 07 D 249:00) |
| D,A | US - A - 4 605 433 (N.R.PEARSON et al.)<br>* Ansprüche 1,10; Spalte 3 *<br>-- | 1,10-13,20-25 | |
| D,A | JOURNAL OF THE AMERICAN CHEMICAL SOCIETY, Band 100, 1978<br>T.DEACON et al. "Reactions of Nucleophiles with Strained Cyclic Sulfonate Esters. Brønsted Relationships for Rate and Equilibrium Constants for Variation of Phenolate Anion Nucleophile and leaving Group"<br>Seiten 2525-2534<br>* Seite 2526 *<br>-- | 28 | RECHERCHIERTE SACHGEBIETE (Int. Cl.4)<br>C 07 D 487/00<br>C 07 D 327/00 |
| A | JOURNAL OF THE AMERICAN CHEMICAL SOCIETY, Band 95, 1973<br>P.CAMPBELL et al. "Reaction of a Six-Membered Cyclic Sulfonate Ester, ß-(2-Hydroxy-3,5-dinitrophenyl)-ethanesulfonic Acid Sulfone, with the Active Site of Papain"<br>Seiten 3735-3741<br>* Seite 3736 *<br>-- | 28 | |

Der vorliegende Recherchenbericht wurde für alle Patentansprüche erstellt.

| Recherchenort | Abschlußdatum der Recherche | Prüfer |
|---|---|---|
| WIEN | 28-06-1989 | PETROUSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung



EP 89810255.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | THE JOURNAL OF ORGANIC CHEMISTRY, Band 40, 1975<br>B.E.HOOGENBOOM et al. "Chemistry of Sulfoacetic Acid Derivatives. III. Reactions of Derivatives of Sulfoacetic Acid, Benzoylmethanesulfonic Acid, and p-Nitrophenylmethanesulfo--nic Acid with Salicyclaldehydes" Seiten 880-883<br>* Seite 881 *<br>---- | 28 | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prufer |
|---|---|---|
| WIEN | 28-06-1989 | PETROUSEK |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82